# EUROPEAN PATENT SPECIFICATION

(11) **EP 0 833 645 B1**
(45) Date of publication and mention of the grant of the patent: **03.09.2003**
(21) Application number: 96918824.2
(22) Date of filing: 20.06.1996
(51) Int. Cl.: A61K 35/80, A61K 31/715, C08B 37/00, A61P 31/22

(54) **ANTIVIRAL AGENTS**
ANTIVIRALE MITTEL
AGENTS ANTIVIRAUX

(30) Priority: 22.06.1995 IL 11426795
(43) Date of publication of application: 08.04.1998
(73) Proprietor: BEN-GURION UNIVERSITY OF THE NEGEV RESEARCH AND DEVELOPMENT AUTHORITY, 84419 Beer-Sheva (IL)
(72) Inventor: ARAD, Shoshana, 84965 Omer (IL); HULIHEIL, Mahmoud, 84428 Beer-Sheva (IL); TAL, Jacov, 84428 Beer-Sheva (IL)
(74) Representative: Boulinguiez, Didier
(86) International application number: IL9600019
(87) International publication number: WO97000689

(56) References cited:
- EP-A- 0 295 956
- US-A- 4 162 308
- BIOCHEMICAL PHARMACOLOGY 47 (12). 1994. 2187-2192, XP000607262 DAMONTE E ET AL: "Antiviral activity of a sulphated polysaccharide from the red seaweed Nothogenia fastigiata."
- J CANCER RES CLIN ONCOL 113 (5). 1987. 413-416, XP000608210 NAKASHIMA H ET AL: "ANTIRETROVIRAL ACTIVITY IN A MARINE RED ALGA REVERSE TRANSCRIPTASE INHIBITION BY AN AQUEOUS EXTRACT OF SCHIZYMENIA -PACIFICA."
- BIOMASS BIOENERGY (1991), 1(2), 83-8, 1991, XP000607263 COHEN, EPHRAIM ET AL: "A closed system for outdoor cultivation of microalgae"

## Description

### Field of the Invention

The invention relates to antiviral agents. More particularly, the invention relates to the use of red microalgal polysaccharides as antiviral materials.

### Background of the Invention

Various algal polysaccharides have been found to possess antiviral activity against different viruses. The potential antiviral activity of algal polysaccharides was first shown by Girber et al., *[Proc. Soc. Exp. Biol. Med. 99, 590-593, 1958]* who observed that polysaccharides extracted from *Gelidium cartilagenium* and carrageenan (from *Chondrus crispus*) afforded protection for embryonated eggs against influenza B and mumps viruses. These algal polysaccharides possessing antiviral activity were identified as highly sulfated polysaccharides. Addition of a polycation such as DEAE-dextran counteracted the inhibitory action of the negatively charged inhibitor.

Since these studies, several biological and synthetic sulfated polyanions such as heparin, has been shown to inhibit the replication of different mammalian viruses. It was found that polyanions like heparin were able to prevent viral infection only when added during the early stages of the infection. On the other hand, others found that algal polysaccharides like carrageenan had no effect on virus attachment or penetration into host cells. It was also found that sulfated algal polysaccharides selectively inhibited human immunodeficiency virus (HIV) reverse transcriptase (R.T.) enzyme and replication *in vitro.* In *in vivo* studies it was found that heparin was able to promote tumor regression in mice.

Others found that a number of natural and synthetic polyanions induced interferon production both *in vitro* and *in vivo*.

EP 295956 deals with non-sulphated antiviral polysaccharides derived from different algae, particularly from seaweed, which are extracted from the cells, and which have a relatively low molecular weight, namely, these described polysaccharides are in the form of cellular extracts and possibly also contain various impurities. The exemplified polysaccharides are described in EP 295956 to have a molecular weight only in the range of between 10³ and 3x10⁶, which are significantly smaller than the new polysaccharides of the present invention. Further, these polysaccharides are said to be used in the treatment of retroviral infections, particularly of AIDS. EP 497341, on the other hand, discloses pharmaceutical compositions for the treatment of viral infections, comprising a combination of a fibroblast growth factor and a sulfated polysaccharide, which are used against viruses such as Herpes simplex virus (HSV) and HIV. The polysaccharide can be of a variety of types, and can, *inter alia*, be derived from a red alga, in which case it has a backbone of the agaroidtype, composed of alternating β(1→ 4)D-galactose and α(1→ 3)L-galactose repeating units. However, it should be noted that one drawback of the known antiviral polysaccharides for example, some of those in EP 497341, are their toxicity and hence some polysaccharides of the type described in EP 497341 do not represent polysaccharides which may be effectively used therapeutically as antiviral agents, due to their toxicity to mammalian cells.

### Summary of the Invention

It has now been found, and this is an object of the present invention, that sulfated polysaccharides derived from red microalgae are effective antiviral agents.

It has further been found, and this is another object of the invention, that red microalgae polysaccharides can be used to inhibit virus replication in host cells.

It has also been found, and this is another object of the invention, that treatment of healthy cells with red microalgae polysaccharides is effective to prevent viral infection.

It is a further object of the invention to provide antiviral compositions based on red microalgae polysaccharide together with known antiviral agents, which are effective to treat a variety of resistant HSV strains, e.g., acyclovir-resistant HSV strains.

It is an object of the invention to provide antiviral compositions containing red microalgae polysaccharides, which overcome the drawbacks of known antiviral polysaccharides. Other objects of the invention will become apparent as the description proceeds.

### Brief Description of the Drawings

- Fig. 1 -: Effect of *Porphyridium sp. polysaccharide* (abbreviated hereinafter as P.spP) on the development of HSV-1 cytopathic effect;
- Fig. 2 -: Effect of P.spP on cell proliferation;
- Fig. 3 -: Effect of P.spP on vero cell infection with HSV-1;
- Fig. 4 -: Effect of P.spP on the development of Varicella Zoster virus (VSV) cytopathic effect;
- Fig. 5 -: Development of HSV-1 cytopathic effect after P.spP removal;
- Fig. 6 -: Addition of P.spP to pre-infected cell cultures;
- Fig. 7 -: Effect of P.spP on production of infectious virus in a single replication cycle; and
- Fig. 8 -: Cytotoxic effects of Carrageenan K and dextran sulphate compared with P.spP.

### Detailed description of the invention

In one aspect, the invention is directed to an antiviral composition, comprising as an active ingredient an antivirally-effective amount of a polysaccharide derived from red microalga or a mixture of two or more red microalgae polysaccharides.

According to one preferred embodiment of the invention, the antiviral composition comprises as an active ingredient an effective infection-inhibiting amount of a polysaccharide derived from red microalgae. While the polysaccharide can of course be obtained from the cell walls by different procedures, e.g., by extraction, it is preferred to employ polysaccharide excreted by the algae into the growth medium, which is essentially a pure form of the polysaccharide. This, apart from other considerations, such as less danger of co-extraction of undesired materials, which exists when extraction procedures are employed, is of course much more convenient and economic from the industrial point of view. Such compositions are useful to treat already infected cells, such as virally infected wounds in mammals, by inhibiting the replication of the virus within the host cell and preventing viral re-infections. Of course, inhibition of the replication of the virus and the prevention of viral re-infections lead to the disappearance of the symptoms of the viral infection.

According to another preferred embodiment of the invention, the antiviral composition comprises as an active ingredient an amount of a polysaccharide derived from red microalgae which is effective to protect against viral infection. According to this embodiment of the invention, healthy cells are treated with the polysaccharide, the presence of which prevents their infection by the virus.

Of course, the antiviral composition of the invention can be provided in any suitable vehicle. Thus, for instance, the antivirally-effective polysaccharide derived from red microalgae can be provided together with pharmaceutically-acceptable vehicles and/or carriers and/or adjuvants. Suitable administration vehicles include, e.g., creams, ointments and solutions and suspensions (liquid forms). However, it should be noted - and this is a substantial advantage of the invention - that the polysaccharide can be used "as is", namely, in its natural form as excreted by the microalgael cells, viz., once it has been isolated from the medium it exists in a slightly viscous form which can conveniently be used without the addition of any carrier or excipient. Of course, whenever desired, be it for a particular. form of application or in order to provide the polysaccharide together with other active materials, the polysaccharide can be formulated in many different ways, as conventional in pharmaceutical practice, and as apparent to the skilled person.

Additionally, the antiviral compositions of the invention may further comprise additional conventional antiviral agents, such as acyclovir, famcyclovir, velacyclovir, idoxuridine, rifluridine, vidarabine, interferons, and the like. It has been found, and this is an object of the invention, that it is convenient to provide antiviral compositions containing both a polysaccharide and a known antiviral agent, e.g., acyclovir. This is because a number of acyclovir-resistant HSV strains exist which, the inventors have found, are effectively treated with the red microalgae polysaccharide. Thus, by providing, e.g., an acyclovirpolysaccharide mixed formulation, all types of HSV infections may be treated without the need to test the patient and to determine whether the particular strain from which he suffers is or is not resistant to acyclovir. In a similar fashion, the polysaccharides of the present invention may also be used to treat infections by other viruses, especially when strains of such viruses have become resistant to conventional agents used in their treatment, the combination of the polysaccharide of the invention and one or more of such conventional agents then being most effective for the treatment of these other viruses in that such a combination would be effective against viruses whether they are resistant or sensitive to the conventional agents.

While the invention is not limited to the treatment or the prevention of infections caused by any particular virus, one useful and widespread virus for which is it particularly useful is the Herpes simplex virus (type 1 and type 2). Other viruses may be treated according to the present invention, such as, for example, Varicella zoster virus (VZV), respiratory syncitial virus (RSV), Murine Leukemia and Sarcoma viruses (MuLV and MuSV).

Red microalgae are well known in the art, and will be readily recognized by the skilled person. The polysaccharides of the invention obtained in the form of polysaccharides excreted from different red microalgae vary in effectiveness, although they are all active. A preferred microalga from which the antiviral polysaccharide is derived is selected from among *Porphyridium sp., P. aerugineum*, and *R. reticulata*. The microalgae can be grown in any conventional way well known to the skilled person, e.g., by the method and apparatus described in EP 576870 of the same applicants herein.

Therefore, in one aspect the invention is directed to the use of a composition comprising as an active ingredient an antivirally-effective amount of a polysaccharide derived from red microalgae, for the treatment or prevention of viral infections.

In another aspect, the invention is directed to the use of red microalgae for the preparation of an antiviral medicament.

The monosugar composition and the stoichiometry of various moities of the polysaccharides to be used may be determined by means of acid hydrolysis under a variety of conditions, followed by chromatographic techniques. The main monosaccharides to be found in the polysaccharides of the red microalgal species are Xylose, Glucose and Galactose.

The summary of the characteristics of the red microalgal polysaccharides exemplified in the description to follow, is given in Table I below.

**Table I**

| **Chemical composition of the polysaccharides from various red microalgae.** | | | |
|---|---|---|---|
| **Sugar (%)** | **P.spP** | **R.rP** | **P.aP** |
| *MEO*-pentose | 1.2 | 0.2 | 1.5 |
| Rhamnose | 0.1 | 0.3 | 17.2 |
| Aarabinose | 0.8 | 2.2 | 5.8 |
| Xylose | 41.2 | 25.2 | 21.06 |
| *MeO*-galactose | 1.5 | 2.9 | 1.0 |
| Mannose | 1.1 | 0.5 | 5.8 |
| Glucose | 15.4 | 20.9 | 21.8 |
| Galactose | 29.4 | 43 | 15.7 |
| Dimethyl galactose | 3 | - | - |
| Glucuronic Acid | 7.3 | 4.8 | 9.8 |
| Sulfate | 2.8 | 2.04 | ≤ 0.5 |
| Legend: P.spP = *Porphyridium sp. polysaccharide*; P.aP = *P. aerugineum polysaccharide;* and R.rP = *R. reticulata polysaccharide*. | | | |

The above-described red microalgal polysaccharides may be further characterized by having enzymatic degradation while applying an extract of a dinoflagellate isolated from open culture of *Porphyridium* cells, as demonstrated in Simon et al., *J. Phycol*. 28, 460-465 (1992), or a mixture of soil bacteria or a dinoflagellate as was demonstrated by Arad (Malis) et al. in *Phytochem*., 32, 2, 287-290 (1993).

These polysaccharides may also be characterized by their physical properties having a typical solubility of 10 to 20 gr./liter, the viscosity of such a solution being 25 to 40 cp. and molecular weight of 5 - 7 x 10⁶. They are also characterized by their stability to wide range of pH, of temperatures, of osmolalities, various saltiness and resistance to biodegradation. No known carbohydrolases cleave the polysaccharides of the invention, and therefore it was necessary to turn to soil bacteria or a dinoflagellate, as discussed above, in order to achieve biodegradation.

The temperature resistance of the polysaccharide makes it possible to carry out its autoclave sterilization without adversely affecting its antiviral activity. This, as will be understood by the skilled person, is a substantial advantage of the polysaccharides of the invention, from an industrial and practical viewpoint.

The invention will now be illustrated with reference to the following examples. The examples, materials and methods employed are not intended to limit the invention in any way, but rather are provided for the purpose of illustration only.

### MATERIALS AND METHODS

The following materials and methods were used throughout the following examples.

### Cells and viruses.

Green monkey kidney cells (Vero cells) were grown in RPMI medium supplemented with 10% newborn calf serum and containing antibiotics (penicillin, streptomycin and neomycin).

Herpes simplex viruses type 1 and 2 (HSV-1 and HSV-2) were used and grown on Vero cells. The virus concentration was estimated by plaque assay.

### Preparation and Purification of Microalgal Polysaccharides.

Briefly, polysaccharides were prepared from red microalgae *(Porphyridium sp*., *"P.sp"; Porphyridium aerugineum, "P.a" and Rhodella reticulata, "R.r")* which were grown under conditions optimal for their growth. The algae were harvested at the stationary phase of growth, the cells were separated from the growth medium by centrifugation after which the supernatant was submitted repeatedly to cross-flow filtration, first to remove salts and then to increase concentration.

More specifically, the following is the detailed procedure for growing *Porphyridium* sp., this procedure also being useful for the above other microalgal species with minor modifications to meet the specific optimal requirements of each different species as is known to those skilled in the art:

*Porphyridium* sp. was grown in artificial seawater (ASW) according to Jones et al. (Jones, R.F., Speer H.L. and Kury, W. (1963), Physiol. Plant 16, 636-643). The ASW was prepared from the following component solutions:
Macroelements Solution

| | |
|---|---|
| NaCI | 27 g/l |
| MgSO₄·7H₂O | 6.6 g/l |
| MgCl₂·6H₂O | 5.6 g/l |
| CaCl₂·2H₂O | 1.5 g/l |
| KNO₃ | 1.0 g/l |
| KH₂PO₄ | 0.07 g/l |
| NaHCO₃ | 0.04 g/l |

Microelements Solution

| | |
|---|---|
| ZnCl₂ | 4.0 mg/100 ml |
| H₃BO₃ | 60.0 mg/100 ml |
| CoCl₂ 6H₂O | 1.5 mg/100 ml |
| CuCl₂·2H₂O | 4.0 mg/100 ml |
| MnCl₂·4H₂O | 40.0 mg/100 ml |
| (NH₄)Mo₇O₂₄·4H₂O | 37.0 mg/100 ml |

Iron Solution
240 mg FeCl₃ was dissolved in 100 ml Na₂ EDTA (0.05 M, pH 7.6).
Tris Buffer
One molar stock buffer solution of pH 7.6 was prepared with 121.1 g/l Trizma HCl and 27.8 g/l Trizma base.

For the preparation of one liter ASW, there was added to the above macroelement solution 1 ml of the microelement solution, 1 ml of the iron solution and 20 ml of the tris buffer solution, to provide an ASW of pH 7.6.

The *Porphyridium* sp. cultures in the ASW were initiated indoors as previously described (Adda, M., Merchuk, J. G. and Arad (Malis), S. (1986) Biomass 10, 131-140). The culture was then transferred outdoors to polyethylene sleeves and diluted with ASW to 8-10⁶ cells/ml. The culture was grown in a batch regime having logarithmic growth for 4-5 days at which stage it reached a stationary phase of growth in which the growth medium was enriched with the polysaccharide of the invention. As the algae were grown in such a batch mode, nutrients were not supplied during the growth period. 15-30 days after initiation of the culture, the culture was then centrifuged and the supernatent, enriched with the polysaccharide of the invention (i.e., the polysaccharide having been excreted from the cells), was then separated from the pelleted cells and treated further as noted below. It should be noted that during the growth of the culture, the following parameters were continually monitored: cell number, biomass, amount of polysaccharide in the medium (excreted from the cells) and the viscosity.

The above-mentioned supernatent enriched with the excreted polysaccharide was exposed to cross-flow filtration using a hollow fiber microfiltration cartridge of filtration area 2.1 m², pore size 0.45 µ, fiber internal diameter of 1 mm and housing identifier of size 55.

The polysaccharides so obtained were used throughout the following examples.

### Effect of Algal Polysaccharides on Cell Proliferation.

Vero cells were seeded at an average of 5 x 10⁵ cells per plate in 9.6 cm² plates. They were fed with RPMI medium containing 10% newborn calf serum in the presence of various concentrations of polysaccharides, and incubated at 37°C. The medium was replaced every three days with a fresh medium containing the appropriate concentration of polysaccharide. Each day, cells of three plates from each treatment were trypsinized, counted with a Neubauer hemacytometer and the mean value was calculated.

### Estimation of Cytopathic Effect (CPE).

Monolayers of vero cells were infected with HSV in the presence of various concentrations of polysaccharides. After several virus replication rounds (24 to 48 hours of incubation at 37°C) in the presence of the polysaccharide, the cytopathic effects (referred to hereinafter as "CPE") defined by cell degradation, granulous and deformed cell nuclei, rough cell membranes, partial or complete loss of cell anchorage to the plate surface, and developing of intracellular vacuoles, were examined in cell cultures under a phase-contrast microscope. The development of the CPE was examined every 24 hours up to the end of the experiment.

### Cell Infection.

Vero cell monolayers were incubated with HSV in 2% serum at 37°C for 2 hours. Then unadsorbed virus particles were removed, a fresh medium containing 2% serum was added and cell monolayers were incubated at 37°C.

### Plaque Assay.

After an adsorption period of 2 hours at 37°C, the unadsorbed virus was removed and an overlay of medium containing 0.6% agar and 2% calf serum was added. All monolayers were incubated at 37°C in a humidified atmosphere of 5% CO₂ in air for several days (4 - 7 days) until cytopathic effect was observed. The overlay was then removed, the cell monolayer was fixed with formol/saline (10% formalin in saline), the cells were stained with crystal violet and plaques were counted.

### Example 1

### Inhibitory Effect of Red Microalgal Polysaccharides on HSV-1 and HSV-2 Cytopathic Effects (CPE) in Vero Cells.

The cells were infected with HSV-1 or HSV-2 in the presence of various concentrations of three variations of algal polysaccharides. CPE₅₀ represents concentration of polysaccharide that offers 50% protection of the cytopathic effect. The results indicate a significant inhibition of HSV-1 and HSV-2 infection caused by the use of these polysaccharides (Table II). The best results were obtained while using P.spP, which did not exhibit any cytotoxic effects even at concentrations 100 times greater than required to obtain the CPE₅₀ protection.

**Table II**

| algal polysaccharide | HSV strain | CPE₅₀ (µg/ml) |
|---|---|---|
| P.spP | HSV-1 | 1 |
| | HSV-2 | 5 |
| R.rP | HSV-1 | 10 |
| | HSV-2 | 20 |
| P.aP | HSV-1 | 20 |
| | HSV-2 | 50 |

### Example 2

### Effect of P.spP on the development of HSV-1 Cytopathic Effect (CPE).

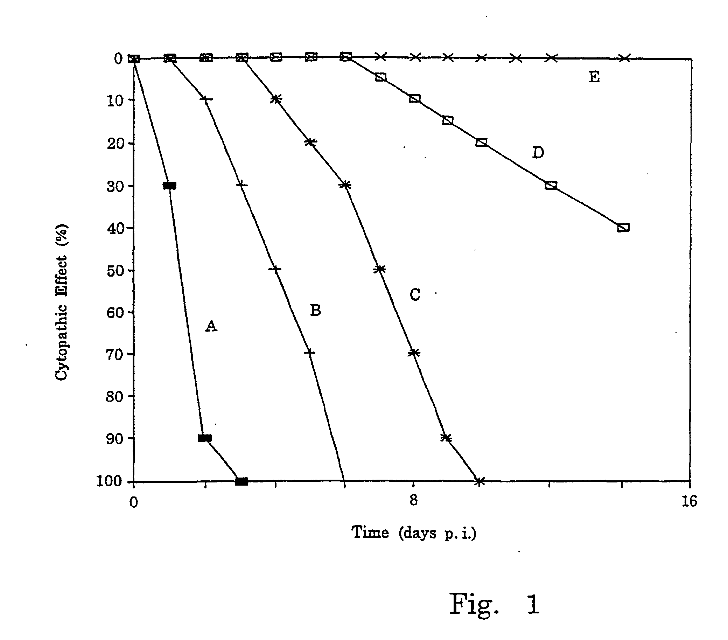

A series of experiments was conducted in which various concentrations of P.spP were used, in order to determine the effect of P.spP concentration on the development and progress of CPE in vero cells infected with 1 multiplicity of infection (referred to hereinafter as "moi") of HSV-1. The results obtained are shown in Fig. 1. Curve "A" demonstrates the cytopathic effect in the absence of P.spP. Curve "B" refers to the use of 1 µg/ml of P.spP; curve "C" refers to 10 µg/ml of P.spP; curve "D" refers to 50 µg/ml of P.spP, and "E" refers to 100 µg/ml of P.spP. It appears that as a result of infecting the cells with HSV-1 in the presence of increasing concentrations of P.spP there is a delay in the appearance of the CPE. In addition, the kinetics of CPE development and progress were much slower as a result of increasing P.spP concentration. It can be seen that 100 µg/ml of P.spP fully protected against the destruction of the cell monolayer by HSV-1 during the period of the experiment (2 weeks). This concentration of P.spP had no deleterious effects on uninfected cells, as demonstrated in Example 3.

### Example 3

### Effect of P.spP on Cell Growth.

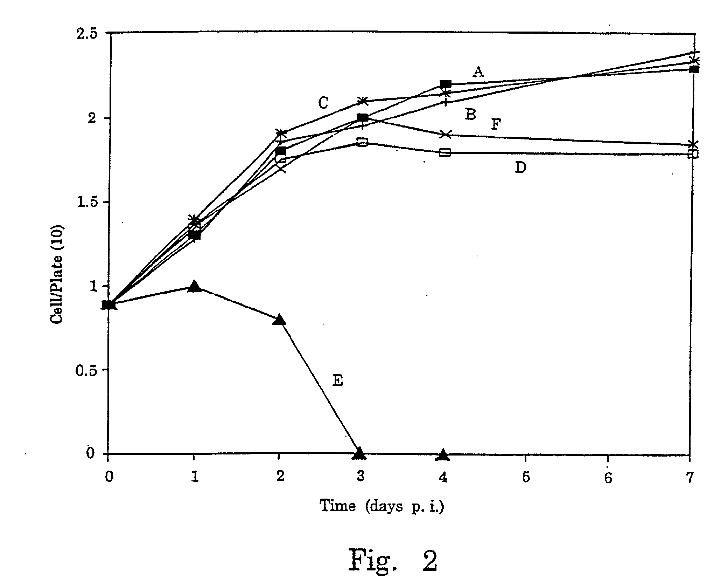

In order to test possible cytotoxic effects of P.spP on cell culture, its effect on cell morphology and cell proliferation was examined. Vero cells were seeded at low concentration and treated with various concentrations of P.spP for 7 days as explained in Example 1. Fig. 2 illustrates vero cells which were seeded at a concentration of 0.9x10⁶ cell per 9.6 cm² plate. These cells were incubated at 37°C in the absence (curve A) or presence of 25 µg/ml (curve B), 250 µg/ml (curve C) or 1,000 µg/ml (curve D) of P.spP. Other Vero cells monolayers were infected with HSV-1 at 1 moi in the absence (curve E) or presence (curve F) of 1,000 µg/ml of P.spP. Microscopic observations showed that there was no effect on cell morphology even when 1,000 µg/ml of P.spP was used. Fig. 2 shows that P.spP has no effect on the proliferation of Vero cells even when a concentration of 250 µg/ml of P.spP was used. Cells treated with 1,000 µg/ml of P.spP grew at control levels during the first 3 days, then stopped their growing. Moreover, HSV-1 infected cells were able to proliferate as uninfected cells when they were treated with P.spP from the beginning of infection.

### Example 4

### Effect of P.spP on Primary Infection.

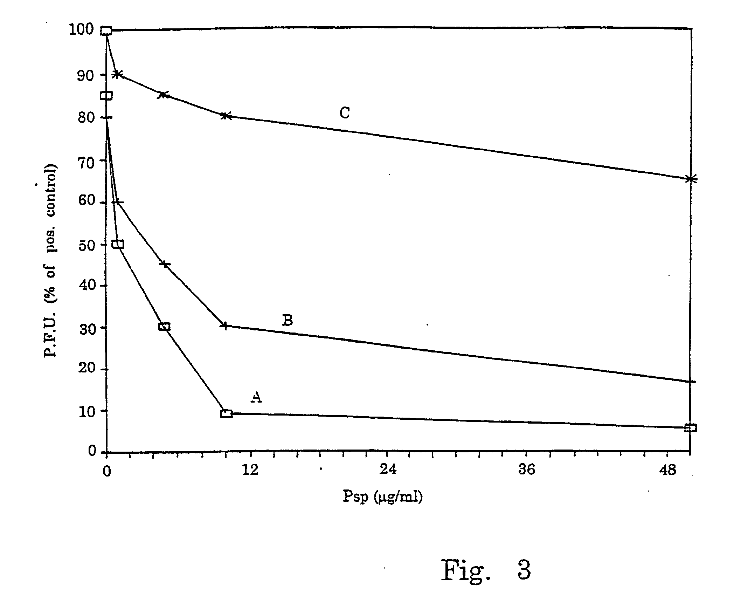

Confluent Vero cell cultures were infected with 1 moi of HSV-1 in the presence of various concentrations of P.spP. The number of primarily infected cells was determined by the plaque assay. Fig. 3 (curve A) shows Vero cells which were infected with 1 moi of HSV-1 in the presence of various concentrations of P.spP. Pfu (plaque forming units) were evaluated by the standard plaque assay. The results (Fig. 3 - curve A) showed that 10 µg/ml of P.spP caused 90-95% protection of the plaques formation. 50% protection of the plaques formed by HSV-1 corresponds to 1 µg/ml of P.spP.

### Example 5

### P.spP-Virus Interaction.

5 Moi of HSV-1 were incubated with various concentrations of P.spP at 37°C for 30 minutes. Then the effect of free polysaccharides was reduced either by (1) making serial dilution of the mixture with medium containing 2% serum before infection, (2) or by precipitating the virus - P.spP mixture through 20% sucrose layer, making serial dilutions of the precipitated virus and infecting Vero cells. The effect of P.spP on HSV-1 infection was estimated by the number of plaques formed as determined by the plaque assay. There was a strong inhibition in plaque formation either when high dilution up to 10⁻³ of the virus-P.spP mixtures was made (Fig. 3 - curve B) or when this mixture was precipitated through sucrose layers (Table III). In addition, it can be seen that at a concentration of 10 µg/ml of P.spP, about 70-80% inhibition in plaques formation was obtained in comparison to 90-95% inhibition when the same concentration of P.spP was added at the time of infection (Fig. 3 - curve A).

**Table III**

| Inactivation of HSV-1 by P.spP (FFU/ml) | | | | | |
|---|---|---|---|---|---|
| Dilution of Virus-P.spP or virus-medium mixture | medium virus mixture | P.spP conc in P.spP-virus mixture (µg/ml) | | | |
| | | 1 | 5 | 50 | 100 |
| Undiluted | *** | *** | ** | * | * |
| 0.01 | 600 | 450 | 200 | 150 | 70 |
| 0.001 | 70 | 50 | 30 | 18 | 6 |
| 0.0001 | 12 | 8 | 4 | | |
| *, **, *** represent various degrees of uncountable number of PFU/ml. | | | | | |

### Example 6

### P.spP-Cell Interaction.

In order to test possible interaction between P.spP and host cells, which may interfere with virus adsorption, vero cells were incubated with a medium containing different concentrations of P.spP at 37°C for two hours. At the end of incubation, unbound P.spP was removed and cell monolayers were washed three times with PBS. Then these cell cultures were infected with 1 moi of HSV-1 and the number of plaques formed was determined by plaque assay. The results (given in curve C, Fig. 3) show a weak inhibition of viral infection of those P.spP pretreated cell-cultures.

### Example 7

### Effect of P.spP on the development of Varicella Zoster Virus (VZV) CPE.

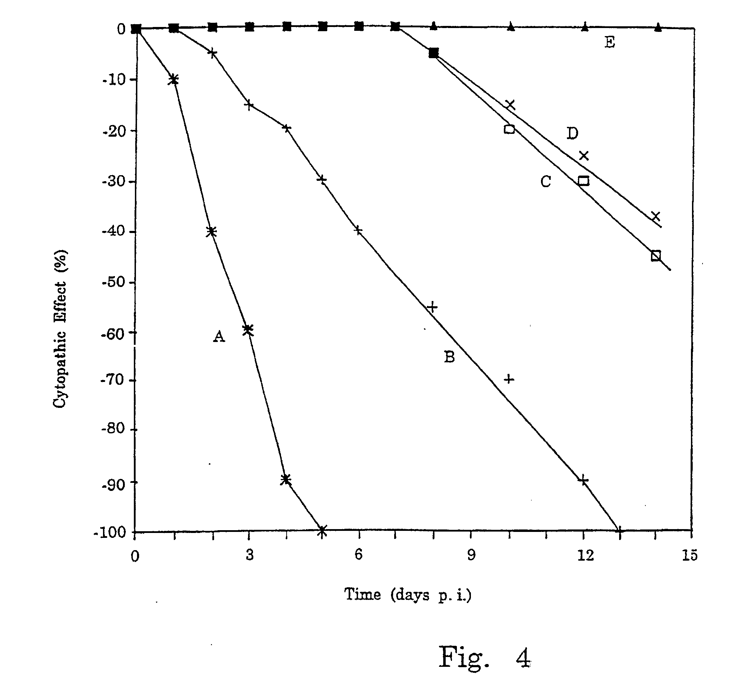

A series of experiments was conducted in which various concentrations of P.spP
were used, in order to determine the effect of P.spP concentration on the development and progress of CPE in Vero cells infected with 1 moi of *Varicella Zoster Virus* (VZV). The results obtained are shown in Fig. 4. The cytopathic effect of VZV is measured in the absence (curve A) or presence of 0.1 µg/ml (curve B), 1 µg/ml (curve C), 100 µg/ml (curve D) or 1,000 µg/ml (curve E) of P.spP. It appears that as a result of infecting the cells with VZV in the presence of increasing concentrations of P.spP, there is a delay in the appearance of the CPE similarly to the phenomenon noted with the HSV-1, as explained in Example 2.

### Example 8

### Effect of P.spP Removal on the Development of HSV-1 CPE.

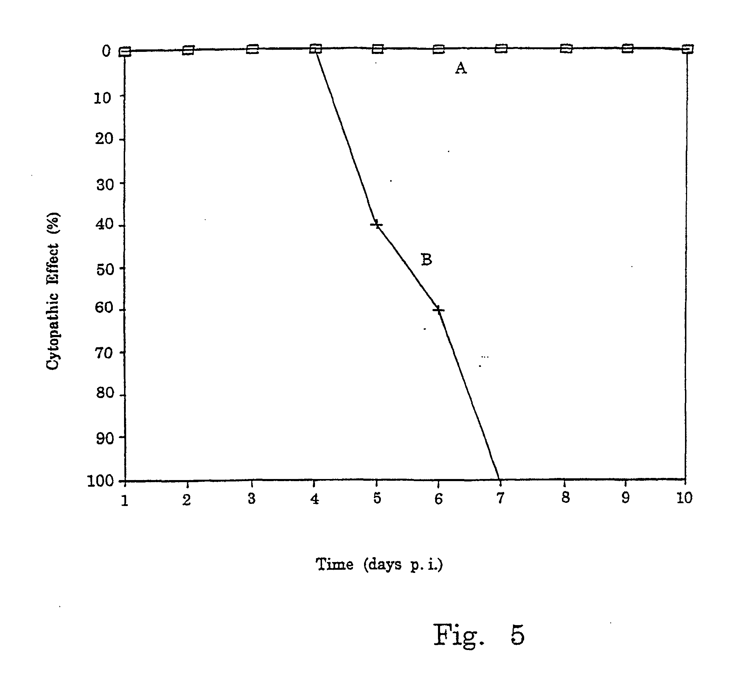

Vero cell monolayers were infected with 0.1 moi of HSV-1 in the presence of 100 µg/ml of P.spP. After two hours of infection, unadsorbed virus Particles were removed, and cell monolayers were washed three times with PBS and fresh medium supplemented with (curve A, Fig. 5) or without (curve B, Fig. 5) P.spP was added. Cell cultures were examined for appearance of CPE by inverted microscope observation. The results presented in Fig. 5 showed that monolayers which were treated with P.spP up to the end of the experiment, exhibited full protection against HSV-1 CPE development, whereas in monolayers treated with P.spP only at the time of infection, there was a delay of 4 days in the appearance of CPE.

These results could be explained by the possibility that some of the infecting virus particles succeeded to infect cells in the presence of P.spP. Continuous treatment with P.spP postinfection inhibited the replication of the viruses inside the host cells.

### Example 9

### Addition of P.spP to pre-infected Cell Cultures.

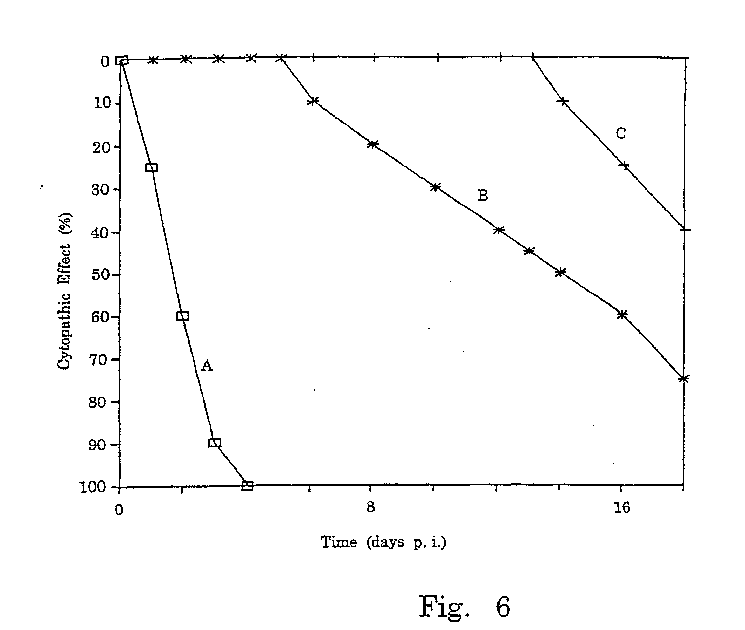

Vero cell monolayers were infected with 0.1 moi (curves A and B, Fig. 6) or 1 moi of HSV-1 (curve C, Fig. 6) without treatment with P.spP. At the end of the infection, monolayers were washed three times with PBS and fresh medium with (curves B and C, Fig. 6) or without (curve A) 100 µg/ml of P.spP was added. Monolayers were examined each day for appearance and development of CPE.

The results indicate that P.spP fully prevented the appearance of CPE in cultures infected with low moi (curve C, Fig. 6). In some of those cultures P.spP was removed 10 days post-infection, and cultures were supplied with fresh medium containing 2% serum without P.spP. Three days after P.spP removal, CPE appeared (curve C, Fig. 6). In cultures which were infected with high moi, there was a delay of 4 days in the appearance of CPE as a result of treatment with P.spP (curve B, Fig. 6). Also, the development rate of CPE in these cultures was very slow, compared to P.spP untreated cultures (curve A, Fig. 6).

### Example 10

### Effect of P.spP on HSV-1 Production in a single Round of Replication.

Vero cell monolayers were infected with 1 moi of HSV-1. At the end of the infection, virus excess was removed, cell cultures were washed three times with PBS and supplied with fresh medium containing various concentrations of P.spP. After 24 hours of incubation at 37°C, the medium was removed, cell cultures were washed three times with PBS, cells were collected and disrupted by three freeze-thaw cycles. Cell debris were precipitated and infectious viruses produced were estimated by the standard plaque assay.

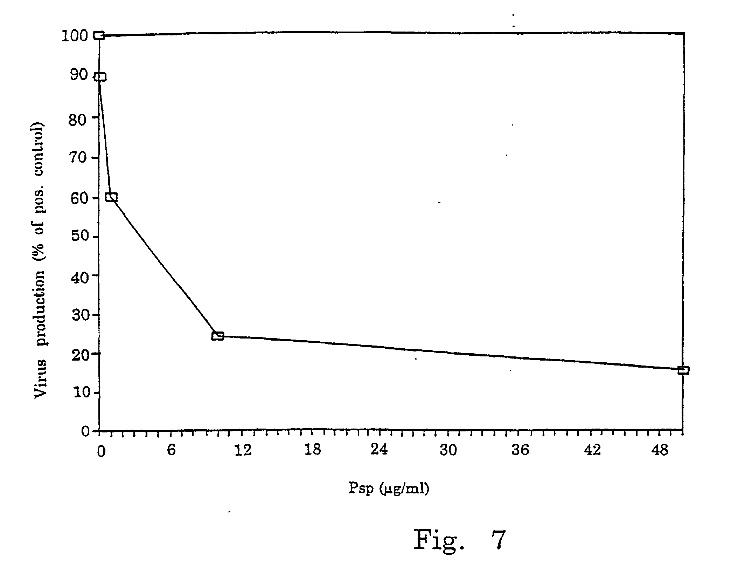

The results shown in Fig. 7, which are presented as a percentage of positive control (P.spP untreated cells) indicate that as little as 1 µg/ml of P.spP profoundly inhibits the production of infectious HSV-1 during a first single cycle of replication.

The results indicate that P.spP can inhibit HSV-1 replication also by affecting its production inside the host cells.

### Example 11

### Cytotoxic effect of various Polysaccharides on Vero cells.

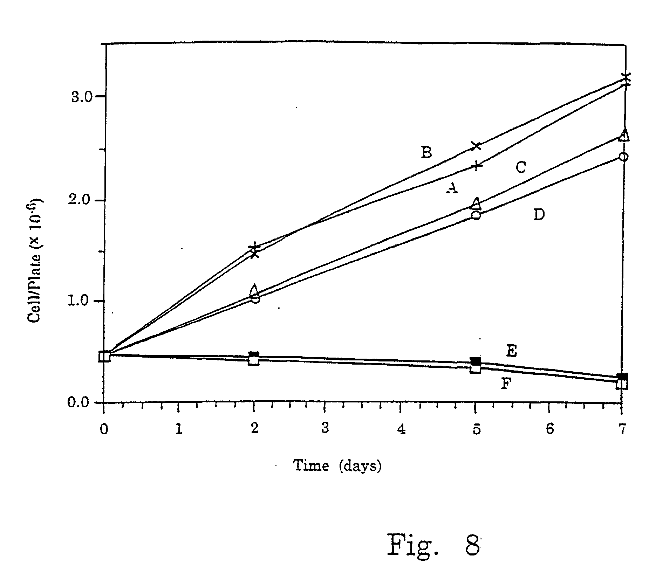

Vero cells were seeded at a concentration of 4 x 10⁵ cells per 9.6 cm² plate. These cells were incubated at 37°C in the absence (curve A, Fig. 8.) or presence of 100 µg/ml of P.spP (curve B), 1 µg/ml(curve C) and 10 µg/ml (curve E) of dextran sulphate, 1 µg/ml (curve D) and 10 µg/ml (curve F) of Carrageenan K. The cells were trypsinized and counted at various intervals of time after the initial treatment with the polysaccharides.

As was clearly demonstrated in Example 2 (Fig. 2), a substantial antiviral effect was obtained while using the P.spP at a concentration of 50 µg/ml or higher. However, when using Carrageenan K and dextran sulphate such concentrations of 50 µg/ml or higher could not be used due to their toxic behavior, even at concentrations as low as 10 µg/ml, as demonstrated in Fig. 8. Thus, while Carrageenan K and dextran sulphate have a similar (low) antiviral activity as P.spP at low concentrations (e.g., 1 µg/ml or less), both Carrageenan K and dextran sulphate cannot however be used at higher antivirally effective concentrations because they are toxic to cells at concentrations greater than about 1 µg per ml. This therefore demonstrates the superiority of P.spP of the invention in that it can be used without toxicity to cells even at concentrations of more than 100 µg per ml.

### Example 12

### Effect of P.spP on HSV-1 Infection of Rabbit Eyes

### Procedure and experiments

Eyes of 1.5 kg rabbits were infected with HSV-1 by scratching the cornea, with a syringe needle, and applying 0.1 ml of the virus suspension (10⁶Pfu/ml) to the scratched area. Appropriate eyes were treated with 1000 µg/ml of *Porphyridium* sp. polysaccharide (P.spP), in the form of drops, twice a day up to the end of the experiment (3-4 weeks).

The following experiments were carried out:
1) Control eyes - Uninfected eyes treated twice a day with phosphate buffered saline (PBS).
2) P.spP - Uninfected eyes treated twice a day with P.spP .
3) Infected eyes - Eyes were infected with the virus without treatment with polysaccharide.
4) Infected and P.spP treated eyes - Eyes were infected and after 15 minutes they were treated with the polysaccharide. The treatment was continued up to the end of the experiment (3-4 weeks) twice a day.
5) P.spP treatment of preinfected symptomatic eyes - Infected eyes with clinical symptoms were treated with P.spP 5-6 days post infection.

### Results:

1) No toxic effects were observed in eyes treated with P.spP only.
2) Eyes infected with HSV-1 (without polysaccharide treatment) showed clinical symptoms (Keratitis with secretions) 5-6 days post infection.
3) All animals with infected eyes (without treatment with the polysaccharide) died 2-3 weeks post infection.
4) Eyes which were infected and treated with the polysaccharide 15 minutes postinfection didn't show any clinical symptoms. The animals so treated survived.
5) Eyes displaying HSV-1 symptoms were treated with the polysaccharide 5-6 days postinfection. All clinical symptoms disappeared and the animals remained alive for a longer period compared with untreated animals. Death occurred at around 4 weeks after infection.

### Example 13

### Effect of P.spP on the Development of HSV-1 Infection in Newborn Rats

Newborn rats (three days old) were infected with 0.1 ml of 10⁷ Pfu/ml of HSV-1 by subcutaneous (s.c.) injection or by scratching (scr). Thirty minutes after infection, the animals were injected s.c. into the infected (s.c.) area with 0.2 ml of P.spP. In the case of scratching the P.spP was added to the scratched area. The treatment was repeated twice a day up to the end of the experiment (death of the animals). Polysaccharide concentrations of 100-1000 µg/ml caused a significant delay in the appearance and development of cutaneous symptoms and a delay in the death of the animals (Table IV).

**Table IV**

| **Inhibitory effect of P.spP on the development of HSV-1 infection in newborn rats** | | | | | | | | |
|---|---|---|---|---|---|---|---|---|
| | **P.spP concentration (µg/ml)** | | | | | | | |
| | **0** | | **100** | | **500** | | **1000** | |
| | **scr.** | **s.c.** | **scr.** | **s.c.** | **scr.** | **s.c.** | **scr.** | **s.c.** |
| Days until the appearance of skin lesions | 3 | 3 | 5 | 5 | 6 | 7 | 6 | 6 |
| Days to death | 7 | 7 | 10 | 10 | 11 | 12 | 12 | 12 |

### Example 14

### Effect of P.spP on Additional Viruses

The general procedures set forth in Examples 2,4, 5 and 13 were repeated with additional viruses, namely, respiratory syncitial virus (RSV), Murine Leukemia and Sarcoma viruses (MuLV and MuSV). Following viral infection of the cells and treatment with the polysaccharide of the invention the effects of the viruses on the cells were determined by standard tests specific for each type of virus. The results (not shown) indicated that for these viruses as well, their growth and infectivity were inhibited significantly by the polysaccharide of the invention.

### Example 15

### Antiviral Effect of Polysaccharides Extracted from Microalgal Cells (Cellular Polysaccharides)

Porphyridum sp. cells were washed with double distilled water and pelleted by centrifugation. The cells pellet was dissolved with double distilled water and heated to 90°C for 4 hours. The mixture was then centrifuged, supernatant was separated and the amount of polysaccharide contained therein was evaluated. This supernatant contains the so-called cellular polysaccharides, i.e., those within the microalgal cells and hence these are the extracted polysaccharides which probably also contain various other cellular components (impurities), in contrast to the above-mentioned excreted polysaccharides which are essentially in pure form.

The above supernatant obtained was tested as in previous examples and showed good antiviral activity against HSV-1, although such activity was somewhat less than that seen with excreted polysaccharide.

All the above description and examples have been provided for the purpose of illustration, and are not intended to limit the invention, except as defined by the claims. Many modifications can be effected in the various methods and materials described above. For instance, different red microalgae can be employed, such microalgae can be grown under different conditions, and the polysaccharide can be extracted in different ways, or can be recovered as excreted polysaccharide from the growth medium. Furthermore, excreted or extracted polysaccharide, or mixtures thereof, or mixtures of two or more polysaccharides derived from different red microalgae, can be used as such, or together with known antiviral or other pharmaceutically-active agents, additives, carriers or excipients. The polysaccharides can be used according to the invention to treat or prevent infections derived from different viruses in various stages, the said infections affecting different areas and cell types, and different application methods, techniques and vehicles can be used, all without exceeding the scope of the invention.

## Claims

1. An antiviral composition, comprising as an active ingredient an antivirally-effective amount of a red microalga polysaccharide, or a mixture of two or more red microalga polysaccharides.

2. An antiviral composition according to claim 1, comprising as an active ingredient an effective replication-inhibiting amount of a red microalga polysaccharide, or a mixture of two or more red microalgae polysaccharides.

3. An antiviral composition according to claim 1, comprising as an active ingredient a red microalga polysaccharide in an amount effective to protect against viral infection.

4. An antiviral composition according to any one of claims 1 to 3, wherein the antivirally-effective amount of the red microalga polysaccharide is provided essentially free of pharmaceutically-acceptable vehicles and/or carriers and/or adjuvants.

5. An antiviral composition according to any one of claims 1 to 3, wherein the antivirally-effective amount of the red microalga polysaccharide is provided together with pharmaceutically-acceptable vehicles and/or carriers and/or adjuvants.

6. An antiviral composition according to any one of claims 1 to 3, wherein the red microalga polysaccharide is a sulphated polysaccharide.

7. An antiviral composition according to any one of claims 1 to 3, comprising a polysaccharide **characterized by** a solubility of 10 to 20 gr/liter, a viscosity of its solution of 25 to 40 c.p., and a molecular weight of 5 - 7 x 10⁶.

8. A composition according to claim 5, in cream form.

9. A composition according to claim 5, in ointment form.

10. A composition according to claim 5, in liquid form.

11. A composition according to claim 5, further comprising conventional antiviral agents.

12. A composition according to claim 11, wherein the virus is a Herpes simplex virus.

13. A composition according to claims 11 and 12, wherein the conventional antiviral agent is acyclovir.

14. A composition according to claim 1, wherein the virus is a *Varicella Zoster* virus.

15. A composition according to claim 1, wherein the red microalgae is selected from among *Porphyridium sp., P. aerugineum,* and *R. reticulata*.

16. An antiviral compound which is a red microalga polysaccharide, or a mixture of two or more red microalga polysaccharides.

17. A compound according to claim 16, which is selected from P.spP, P.aP and R.rP.

18. Use of a compound according to claim 16 or 17, for the manufacture of a drug for the treatment or prevention of viral infections.

19. A compound according to claim 16 or 17, which is a polysaccharide **characterized by** a solubility of 10 to 20 gr/liter, a viscosity of its solution of 25 to 40 c.p., and a molecular weight of 5 - 7 x 10⁶.

20. Use of red microalgae for the preparation of an antiviral medicament.

21. A process for the manufacturing of an antiviral agent, comprising
cultivating red microalgae in a suitable medium, under conditions suitable to promote cell polysaccharide growth and excretion into said medium, collecting and concentrating the excreted polysaccharide from said medium, and using same as an active ingredient of said antiviral agent.

22. An antiviral agent, whenever prepared by the process of claim 21.

23. A polysaccharide excreted from a red microalgae, for use as an antiviral agent.

## Patentansprüche

1. Antivirale Zusammensetzung, die als aktiven Bestandteil eine antiviral wirksame Menge an Polysacchariden roter Mikroalgen oder einer Mischung aus zwei oder mehreren Polysaccariden roter Mikroalgen enthält.

2. Antivirale Zusammensetzung gemäß Patentanspruch 1, die als aktiven Bestandteil eine wirksame replikationsinhibierende Menge an Polysaccariden roter Mikroalgen oder eine Mischung von zwei oder mehreren Polysacchariden roter Mikroalgen enthält.

3. Antivirale Zusammensetzung gemäß Patentanspruch 1, die als aktiven Bestandteil in wirksamer Menge ein Polysaccharid roter Mikroalgen enthält, um gegen virale Infektionen zu schützen.

4. Antivirale Zusammensetzung gemäß einem der Ansprüche 1 bis 3, wobei die antiviral wirksame Menge der Polysaccharide roter Mikroalgen gänzlich frei von pharmazeutisch anerkannten Vehikeln und/oder Carrieren und/oder Zusatzstoffen bereitgestellt ist.

5. Antivirale Zusammensetzung gemäß einem der Ansprüche 1 bis 3, wobei die antiviral wirksame Menge der Polysaccaride roter Mikroalgen zusammen mit pharmazeutisch anerkannten Vehikeln und/oder Carriern und/oder Zusatzstoffen bereitgestellt ist.

6. Antivirale Zusammensetzung gemäß einem der Ansprüche 1 bis 3, wobei das Polysaccharid roter Mikroalgen ein sulfatiertes Polysaccharid ist.

7. Antivirale Zusammensetzung gemäß einem der Ansprüche 1 bis 3, die ein Polysaccharid, **gekennzeichnet durch** eine Löslichkeit von 10 bis 20 g/l, einer Viskosität seiner Lösung von 15 bis 40 cP und einem Molekulargewicht von 5 bis 7 x 10⁶, enthält.

8. Zusammensetzung gemäß Anspruch 5 in Cremeform.

9. Zusammensetzung gemäß Anspruch 5 in Salbenform.

10. Zusammensetzung gemäß Anspruch 5 in flüssiger Form.

11. zusammensetzung gemäß Anspruch 5, die desweiteren herkömmliche antivirale Wirkstoffe enthält.

12. Zusammensetzung gemäß Anspruch 11, wobei das Virus ein Herpes-simplex-Virus ist.

13. Zusammensetzung gemäß Ansprüchen 11 und 12, wobei der herkömmliche antivirale Wirkstoff Acyclovir ist.

14. Zusammensetzung gemäß Anspruch 1, wobei das Virus ein Varicella-Zoster-Virus ist.

15. Zusammensetzung gemäß Anspruch 1, wobei die rote Mikroalge aus *Porphyridium sp., P.aerugineum und R. reticulata* ausgewählt ist.

16. Antivirale Zusammensetzung, die ein Polysaccharid roter Mikroalgen oder eine Mischung von zwei oder mehreren Polysacchariden roter Mikroalgen ist.

17. Zusammensetzung gemäß Anspruch 16, die aus P.spP, P.aP und R.rP ausgewählt ist.

18. Anwendung einer Zusammensetzung gemäß Ansprüchen 16 oder 17, zur Herstellung eines Medikamentes zur Behandlung oder Prävention viraler Infektionen.

19. Zusammensetzung gemäß Ansprüchen 16 oder 17, die ein Polysaccharid, **gekennzeichnet durch** eine Löslichkeit von 10 bis 20 g/l, einer Viskosität seiner Lösung von 25 bis 40 cP und einem Molekulargewicht von 5 bis 7 x 10⁶, ist.

20. Benutzung roter Mikroalgen zur Herstellung eines antiviralen Medikamentes.

21. Verfahren zur Produktion eines antiviralen Wirkstoffs, durch Kultivierung roter Mikroalgen in einem adäquaten Medium, unter adäquaten Bedingungen, die das Zellpolysaccharidwachstum und die Ausscheidung in das genannte Medium fördern, Sammeln und Konzentrieren der ausgeschiedenen Polysaccharide aus dem genannten Medium und Benutzung desselben als aktiven Bestandteil des genannten antiviralen Wirkstoffs.

22. Antiviraler Wirkstoff, immer wenn er durch das Verfahren in Anspruch 21 hergestellt wurde.

23. Polysaccarid zur Benutzung als antiviralen Wirkstoff, dae von einer roten Mikroalge ausgeschieden wurde.

## Revendications

1. Composition antivirale, comprenant comme ingrédient actif une quantité antivirale efficace d'un polysaccharide de microalgue rouge ou d'un mélange d'au moins deux polysaccharides de microalgue rouge.

2. Composition antivirale selon la revendication 1, comprenant comme ingrédient actif une quantité efficace, inhibant la réplication, d'un polysaccharide de microalgue rouge ou d'un mélange d'au moins deux polysaccharides de microalgue rouge.

3. Composition antivirale selon la revendication 1, comprenant comme ingrédient actif un polysaccharide de microalgue rouge en une quantité efficace pour la protection contre une infection virale.

4. Composition antivirale selon l'une quelconque des revendications 1 à 3, dans laquelle la quantité antivirale efficace du polysaccharide de microalgue rouge est présentée essentiellement sans véhicule et/ou sans support et/ou sans adjuvant pharmaceutiquement acceptable.

5. Composition antivirale selon l'une quelconque des revendications 1 à 3, dans laquelle la quantité antivirale efficace du polysaccharide de microalgue rouge est présentée avec des véhicules et/ou des supports et/ou des adjuvants pharmaceutiquement acceptables.

6. Composition antivirale selon l'une quelconque des revendications 1 à 3, dans laquelle le polysaccharide de microalgue rouge est un polysaccharide sulfaté.

7. Composition antivirale selon l'une quelconque des revendications 1 à 3, comprenant un polysaccharide **caractérisé par** une solubilité de 10 à 20 g/l, une viscosité en solution de 25 à 40 cp, et une masse molaire de 5 - 7 × 10⁶.

8. Composition selon la revendication 5, sous forme de crème.

9. Composition selon la revendication 5, sous forme de pommade.

10. Composition selon la revendication 5, sous forme liquide.

11. Composition selon la revendication 5, contenant en outre des agents antiviraux classiques.

12. Composition selon la revendication 11, dans laquelle le virus est un virus de l'herpes simplex.

13. Composition selon les revendications 11 et 12, dans laquelle l'agent antiviral classique est l'acyclovir.

14. Composition selon la revendication 1, dans laquelle le virus est un virus *Varicella Zoster.*

15. Composition selon la revendication 1, dans laquelle la microalgue rouge est choisie parmi *Porphyridium sp., P. aerugineum*, et *R. reticulata*.

16. Composé antiviral, qui est un polysaccharide de microalgue rouge ou un mélange d'au moins deux polysaccharides de microalgue rouge.

17. Composé selon la revendication 16, qui choisi parmi P.spP, P.aP et R.rP.

18. Utilisation d'un composé selon la revendication 16 ou 17 pour la préparation d'un médicament destiné au traitement ou à la prévention d'infections virales.

19. Composé selon la revendication 16 ou 17, qui est un polysaccharide **caractérisé par** une solubilité de 10 à 20 g/l, une viscosité en solution de 25 à 50 cp, et une masse molaire de 5 - 7 × 10⁶.

20. Utilisation de microalgues rouges pour la préparation d'un médicament antiviral.

21. Procédé de préparation d'un agent antiviral, dans lequel on cultive des microalgues rouges dans un milieu approprié, dans des conditions appropriées pour favoriser le développement et l'excrétion de polysaccharides cellulaires dans ledit milieu, on recueille et on concentre le polysaccharide excrété à partir dudit milieu, et on l'utilise comme ingrédient actif dudit agent antiviral.

22. Agent antiviral préparé par le procédé selon la revendication 21.

23. Polysaccharide excrété à partir d'une microalgue rouge, à utiliser comme agent antiviral.
